# EUROPEAN PATENT APPLICATION

(11) **EP 1 645 251 A2**
(43) Date of publication of application: **12.04.2006**
(21) Application number: 05107302.1
(22) Date of filing: 09.08.2005
(51) Int. Cl.: A61F 5/37

(54) **Orthopaedic abduction cushion, in particular for arm immobilization**

(30) Priority: 25.08.2004 IT VR20040130
(71) Applicant: F.G.P. SRL, 37062 Dossobuono (IT)
(72) Inventor: Turrini, Alberto, 37062 CASTEL D'AZZANO (VR) (IT); Ferrigolo, Moreno, 37062 DOSSOBUONO (VR) (IT)
(74) Representative: Petraz, Gilberto Luigi

(57) **Abstract**

An abduction cushion for orthopaedic use, in particular for supporting the arm following surgery on the rotator cuffs, is the inflatable type and consists of a casing (10) made in two parts, a first inner part made from a layer (11) of pneumatic and inflatable material, and a second outer part made from a layer (12) of a particular type of fabric to which Velcro can be applied.

## Description

### TECHNICAL FIELD

This invention concerns an orthopaedic abduction cushion which can be used after surgery for reconstruction of the shoulder to immobilize the arm and prevent the movement of the rotator cuffs of the shoulder.

This invention refers in fact to an abduction cushion mainly designed to be used by patients who have undergone surgery for reconstruction of the rotator cuffs of the shoulder.

This cushion is the inflatable type and consists of a single piece or several parts joined together and combined in various ways in order to achieve the most appropriate angle to be maintained by the patient for a certain period after this type of operation.

This invention can be applied in the production sector of equipment for orthopaedic use and in particular equipment to maintain the arm joints, and specifically the rotator cuffs, in an immobilized position.

### BACKGROUND ART

It is known that after surgery on the arm and the shoulder, in particular after operations for reconstruction of the rotator cuffs, it is necessary for the patient to wear auxiliary orthopaedic equipment or orthoses, that is to say fixed or removable equipment that allows the limb to be kept in as stable a position as possible for a certain period of rehabilitation.

Following an operation for reconstruction of the rotator cuffs, the arm is normally kept in a blocked position, first totally and then partially, by means of special orthopaedic equipment which is designed to immobilize the joint through structures that allow the arm to be connected to the body.

This orthopaedic equipment is in some cases represented by actual adjustable angle mechanical joints, while in other cases shims or cushions are used.

In both cases the orthopaedic equipment is positioned under the arm and fixed, by means of special fastenings, connecting the arm to the patient's waist or body.

For example, the American patent US 5,487,724 proposes an articulated structure which can be used after surgery for the reconstruction of the rotator cuffs. By means of straps and other fastening means the structure is fixed on one side to the arm and on the other to the patient's waist and body.

This structure allows immobilization of the arm with adjustable angles. It is not, however, widely used due to its complexity and size which makes it somewhat difficult to wear.

This device does in fact have to be fixed to the arm and to various parts of the body, starting from the pelvic area and continuing upwards as far as the shoulders. This obviously limits the patient's movements to a certain extent, not only those of the arm involved in the operation but also of other parts of the body, considerably limiting or preventing for example bending movements at the waist which should instead be unrestrained.

To overcome these problems, solutions are known which instead of the equipment described above envisage the use of orthopaedic abduction cushions, consisting of the use of a body of a certain size and shape made from soft material which is fixed, by means of appropriate straps, to the patient's side and remains positioned and blocked under the arm so that the arm can rest against it.

In this case, however, the problem that has been encountered concerns the difficulty of producing abduction cushions that are able to immobilize the arm at the angle established by the orthopaedic surgeon according to the specific case.

Some types of variable configuration orthopaedic cushions have therefore been produced, with shapes that allow them to be positioned at two different angles, that is to say generally with two possible angles between the arm and the body, for example at 15 and 45 degrees.

This is possible thanks to the particular shape of the cushion, which if positioned in one direction keeps the arm blocked at a certain angle, while if positioned in the other direction it keeps it blocked at another angle.

Problems have been encountered in this case too, mainly due to the impossibility of immobilizing the arm that has undergone reconstruction of the rotator cuffs, it being extremely difficult to position the arm at intermediate angles or angles lesser or greater than those indicated. The patient or the orthopaedic surgeon is therefore often obliged to resort to contrivances in order to achieve the required angle with the cushion.

### DESCRIPTION OF THE INVENTION

This invention proposes to provide an abduction cushion which can be used following surgery for reconstruction of the rotator cuffs, its distinctive feature being that it is made from inflatable material and can therefore eliminate or at least reduce the drawbacks described above.

In fact, being inflatable, the cushion according to this invention can be positioned between the arm and the body, achieving the most appropriate angle by adjusting the pneumatic pressure.

The invention also proposes to provide an abduction cushion that is easy to produce in order to be economically advantageous.

This is achieved by means of an orthopaedic abduction cushion with the features described in the main claim.

The dependent claims describes advantageous embodiments of the invention.

The modular and variable configuration orthopaedic abduction cushion according to the invention thus foresees being made using an inflatable structure, the covering of which is obtained by using two components: a first inner layer in polyurethane or PVC and an outer layer in velvet-like fabric or other material to which Velcro can be applied.

Material to which Velcro can be applied means a honeycomb type of fabric, i.e. a soft fabric of a certain thickness, such as velvet or similar, which can be gripped by the hooked part of the Velcro.

The cushion therefore has the feature of being made from a bi-component material, the inner part in airtight material being joined to the outer fabric part by heat coupling or thermoforming.

This abduction cushion can be produced as a single piece or in several parts joined together with Velcro, the outer covering being made, as described above, from honeycomb fabric suitable for being gripped by the hooked part of the Velcro.

The shape of the inflatable abduction cushion is such that it can be used in different ways according to the inflation pressure, which can be varied as required, with the possibility of obtaining several different angles by which the arm can be immobilized.

If it should be necessary to obtain an extrarotation, a supplementary cushion can be added to the main one, so that the arm can be immobilized at greater angles with respect to the previous one, making it possible to increase the distance between the arm and the body.

The two cushions, the main one and the supplementary one, can be joined together by means of non-permanent adhesive such as Velcro or the like, using the outer part of the fabric to which Velcro can be applied.

The invention in question thus proposes an abduction cushion which can on one hand be produced very easily, considerably limiting production costs, and which on the other hand is versatile due to the possibility of immobilizing the arm in various positions, all at different angles to each other.

The diversification of the immobilization angle is achieved, in the case of normal abduction, by simply inflating the cushion to the pressure appropriate to the required angle, or, in the case of extrarotation, with the addition of a supplementary cushion which can be fixed to the main cushion by means of Velcro.

The main advantages of this solution are:
a) a net reduction in production costs, since the cushion is made very simply, and in the cost of raw materials and production equipment;
b) versatile use, since the variable configuration of the inflatable cushion makes it possible to achieve different ways of immobilizing the arm, according to requirements.

### DESCRIPTION OF THE DRAWINGS

Other features and advantages of the invention will become clear on reading the following description of one embodiment of the invention, provided as a non-binding example, with the help of the accompanying drawings in which:

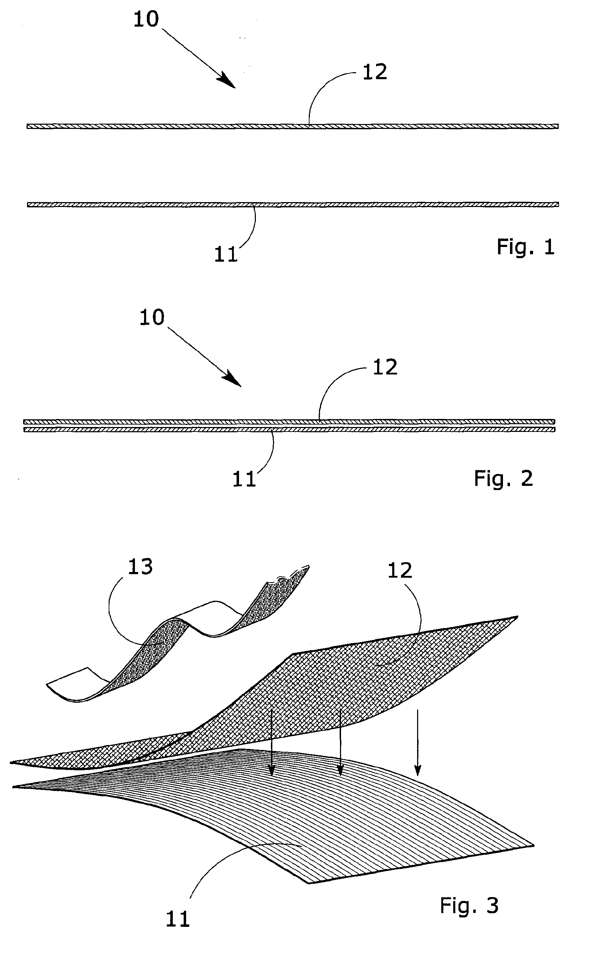

- figure 1 represents a schematic view of the layers which make up the inflatable abduction cushion according to the invention;
- figure 2 shows a schematic view of the two layers joined together;
- figure 3 is a prospective view of the two layers;
- figure 4 is a front view of the inflatable abduction cushion according to the invention;
- figure 5 is a schematic axonometric view;
- figure 6 shows a schematic view of one way of using the abduction cushion according to the invention.

### DESCRIPTION OF ONE EMBODIMENT OF THE INVENTION

The abduction cushion according to the invention represents an orthopaedic device for immobilizing the upper limbs which have been operated on for reconstruction of the rotator cuffs.

According to the invention the abduction cushion is the inflatable type and consists of a casing 10 made in two parts.

A first part of the casing 10, the inner part, consists of a layer 11 made from pneumatic material, such as PVC or another material used for inflatable mattresses.

The second part of the casing 10, the outer part, is formed by a layer 12 made from a particular honeycomb fabric material or fabric to which Velcro can be applied, that is a soft fabric with a certain thickness, such as velvet or the like, which can be gripped by the hooked part of the Velcro 13.

The cushion therefore has the feature of being made from bi-component material, the inner part made from airtight material being joined to the outer part made from fabric by means of heat coupling or thermofusion.

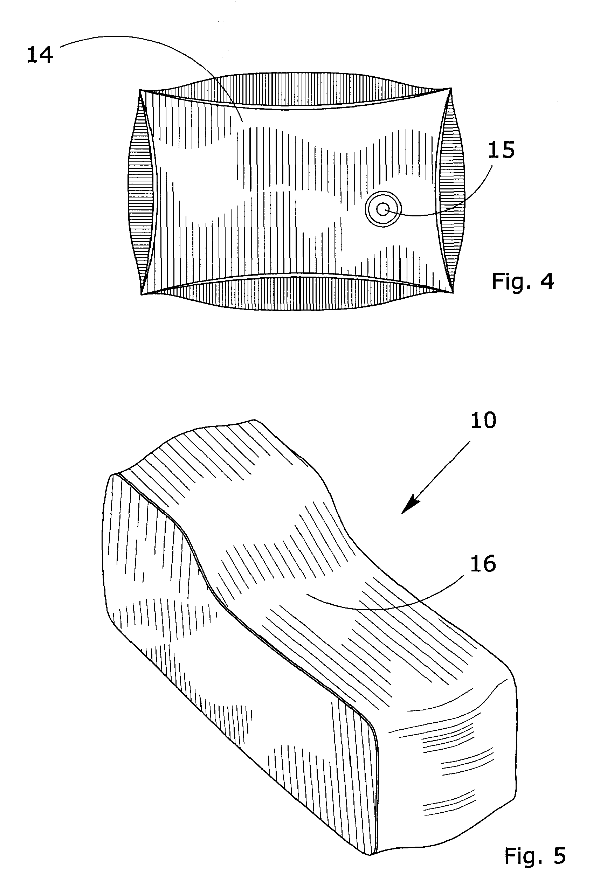

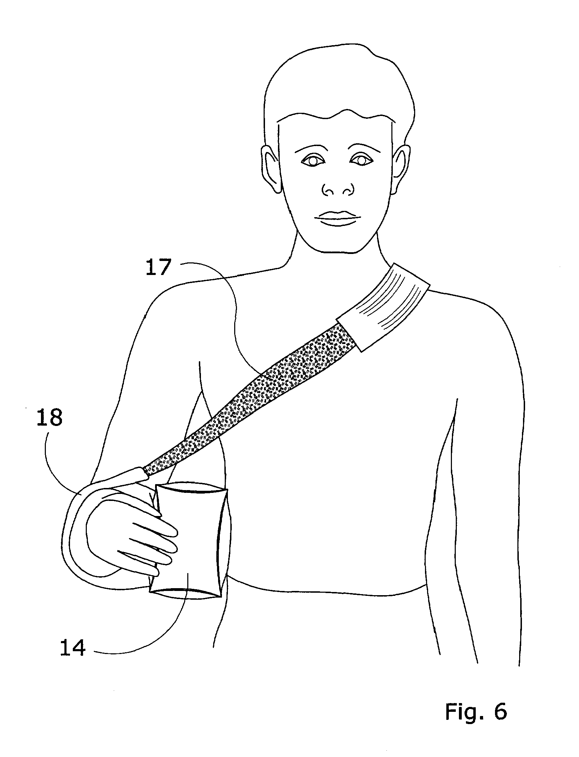

The abduction cushion, indicated overall with 14 in figures 4, 5 and 6, can be made in a single body and in this case it is a cushion with a certain volume and a substantially prismatic or even cylindrical shape, fitted with a valve element 15 for inflating it with air.

The shape of the inflatable abduction cushion is designed to allow different ways of using the cushion according to its position and the inflation pressure, which can be varied as required, with the possibility of obtaining a variety of different angles at which the arm can be immobilized.

If it should be necessary to obtain extrarotation, the main cushion 14 can be supplemented by an additional, generally smaller cushion which, once attached to the main cushion, allows the arm to be immobilized at greater angles with respect to the previous ones, increasing the distance between the arm and the body.

The two cushions, the main one and the supplementary one, can be joined and firmly fixed together to form a practically single body, by means of non-permanent adhesive such as Velcro or the like, applied on the outer parts of the Velcro-applicable fabric.

The cushion can have various shapes according to requirements, even if one of the possible shapes is as shown in figure 5, in which the presence of a curved and shaped side 16 of the cushion can be seen.

This shaped side 16 is the one, in use, which rests against the patient's side, with rounded edges which make it more comfortable to use.

The cushion 14 is fitted with special straps 17 and arm fastening means 18, which can also be fixed by using Velcro, which allow the abduction cushion 14 to be held in place against the patient's waist and body, remaining in position under the arm in which the rotator cuffs have been reconstructed.

According to another advantageous embodiment, the cushion according to the invention with its bi-component type construction method can also be used in other applications in the orthopaedic section, such as for example in the various forms of orthoses applied postoperatively, following trauma and as immobilizers of the arms, the legs and the trunk, if necessary combined with dedicated equipment.

The invention is described above with reference to a preferred embodiment. It is nevertheless clear that the invention is susceptible to numerous variations which are within the framework of technical equivalents.

## Claims

1. An abduction cushion for orthopaedic use, in particular for supporting the arm following surgery on the rotator cuffs, **characterised in that** it is the inflatable type and consists of a casing (10) made in two parts, a first inner part made from a layer (11) of pneumatic and inflatable material, and a second outer part made from a layer (12) of a particular type of fabric to which Velcro can be applied.

2. An abduction cushion for orthopaedic use according to the previous claim, **characterised in that** the inner layer (11) is made from pneumatic material such as polyurethane, PVC or other material used for inflatable mattresses.

3. An abduction cushion for orthopaedic use according to either of the foregoing claims, **characterised in that** it is made from bi-component material, and **in that** the inner part in airtight material is joined to the outer fabric part by means of heat coupling or thermofusion.

4. An abduction cushion for orthopaedic use according to any of the foregoing claims, **characterised in that** it has a certain volume and a substantially prismatic or even cylindrical shape, and is fitted with a valve element (15) for inflating it with air.

5. An abduction cushion for orthopaedic use according to any of the foregoing claims, **characterised in that** it is possible to supplement the main cushion (14) with an additional cushion, also the inflatable type and with the same features as the main cushion, albeit generally smaller, which, once attached to the main cushion, allows the arm to be immobilized at greater angles with respect to the previous ones, increasing the distance between the arm and the body.

6. An abduction cushion for orthopaedic use according to any of the foregoing claims, **characterised in that** these two cushions, the main one and the supplementary one, can be joined and firmly fastened together to form a practically single body, by means of non-permanent adhesive such as Velcro or the like, applied between the outer parts of the Velcro-applicable fabric.
